# EUROPEAN PATENT SPECIFICATION

(11) **EP 4 504 288 B1**
(45) Date of publication and mention of the grant of the patent: **14.01.2026**
(21) Application number: 23717827.2
(22) Date of filing: 29.03.2023
(51) Int. Cl.: A61M 3/02, A61M 39/10, A61M 39/12, A61M 39/20, A61F 5/442

(54) **A CONNECTOR FOR AN IRRIGATION SYSTEM**
VERBINDER FÜR EIN BEWÄSSERUNGSSYSTEM
CONNECTEUR POUR SYSTÈME D'IRRIGATION

(30) Priority: 08.04.2022 DK PA202270192
(43) Date of publication of application: 12.02.2025
(73) Proprietor: Coloplast A/S, 3050 Humlebaek (DK)
(72) Inventor: RADMER, Bo, 3400 Hilleroed (DK); KEISER-NIELSEN, Niels Frederik, 2990 Nivaa (DK); LARSEN, Lars Erup, 3660 Stenloese (DK); FORNÉ, Peter, 2100 Copenhagen OE (DK)
(86) International application number: PCT/DK2023/050064
(87) International publication number: WO 2023/193858

(56) References cited:
- WO-A1-03/030968
- US-A1- 2017 127 888

## Description

The invention relates to a connector for use in an irrigation system, in particular a connector for use in a container for an irrigation system. The invention also relates to a method for de-pressurizing a container in an irrigation system.

Background art can be seen in WO03/030968 and US2017/127888.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 illustrates an anal irrigation system as described herein.
Figures 2A and 2B illustrate a cross-sectional view of a container, with the pressure ratio interior of and exterior of the container indicated.
Figures 3A, 3B, 3C illustrate a cross-sectional view of a container, with the pressure ratio interior of and exterior of the container indicated.
Figures 4 and 4A illustrate a cross-sectional view of an example of a connector in a sealed position. In this position, the container can be pressurized, because air pumped into the container will be prevented from leaving the container through the connector.
Figures 5 and 5A illustrate a cross-sectional view of the connector example of figure 2 in an air-relief position, in figure 3A the eject-mechanism in the form of a lever arm is left out.
Figures 6 and 6A illustrate a cross-sectional view of an example of a connector in a sealed position. In this position, the container can be pressurized, because air pumped into the container will be prevented from leaving the container through the connector.
Figures 7 and 7A illustrate a cross-sectional view of connector example of figure 4 in an air-relief position, figure 5A illustrate a detailed view.
Figures 8 and 8A illustrate a cross-sectional view of the connector example of figure 4, when it is ready to be released from the container.
Figure 9 illustrates a perspective view of an example of a coupling as described herein.
Figures 10A, 10B and 10C illustrate cross-sectional views of the coupling of figure 9 in interaction with a connector. Figure 10A illustrate the connector in an open position, figure 10B illustrate the connector in a sealed position and figure 10C illustrate the connector in a pressure equalizing position.
Figures 11A, 11B and 11B illustrate a lock and release mechanism of a connector.
Figures 12A to 12H illustrate steps in connecting and releasing a connector. All figures are cross-sectional views.
Figures 13A to 13D illustrate steps in connecting and releasing a connector.

### Detailed Description

Examples relate to an anal or stomal irrigation system comprising
- a container configured for containing irrigation liquid and configured for being pressurized,
- a pump configured for pumping air into the container so as to displace the liquid from the container,
- a connector configured for connecting a tube to the container, the connector comprising an inner lumen extending axially through the connector
- the container comprising a coupling comprising at least one seal and a venting hole
   wherein the connector is configured to interact with the coupling to provide the connector with
- a first connected position in which the container can be pressurized by air being pumped into an air-filled portion of the container, and wherein the connector provides a liquid flow path from the container, through the inner lumen of the connector and to the exterior of the container, and
- a second air vented position, wherein an air-flow path is established from the air-filled portion of the container and through the venting hole in the coupling either
   ∘ distally of the connector, or
   ∘ to the exterior of the container, or
   ∘ both distally of the connector and to the exterior of the container.

Examples further relate to a method for de-pressurizing a pressurized container in an anal or stomal irrigation system as described above, the method comprising the connector being transitioned from a first connected position to a second air-vented position, whereby an air-flow path is established from the air-filled portion of the container and through the venting hole in the coupling, thereby de-pressurizing the connector.

Examples relate to an anal or stomal irrigation system comprising
- a container configured for containing irrigation liquid and configured for being pressurized,
- a pump configured for pumping air into the container so as to displace the liquid from the container,
- a connector configured for connecting a tube to the container, the connector comprising an inner lumen extending axially through the connector and a bulge on an exterior surface of the connector
- the container comprising a coupling comprising at least one seal and a venting hole
wherein the bulge of the connector is configured for cooperating with the at least one seal on the coupling such that the bulge and seal in cooperation are configured for being axially aligned and for sealing an interface between the connector and the coupling in a first connected position, and where the bulge and seal are configured for being axially displaced in a second air-vented position thereby establishing an air-flow path from an air-filled portion of the container through the venting hole of the coupling and through the interface between the coupling and the coupling and to the exterior of the container.

Examples relate to an anal or stomal irrigation system comprising
- a container configured for containing irrigation liquid and configured for being pressurized,
- a pump configured for pumping air into the container so as to displace the liquid from the container,
- a connector configured for connecting a tube to the container, the connector comprising an inner lumen extending axially through the connector
- the container comprising a coupling comprising a first seal and a venting hole

wherein the coupling comprises a second seal axially displaced with respect to the first seal and the venting hole, such that the venting hole is positioned axially between the first seal and the second seal,
wherein the coupling is configured for being compressed in an axial direction into two configurations,
   - a first sealed configuration, wherein the first seal and the second seal are displaced in a radially inwards direction, such that the first seal and the second seal are in sealing contact with an exterior surface of the connector, thus providing a sealed configuration of an interface between the connector and coupling, and
   - a second air-vented configuration, wherein the first seal is displaced in a radially inwards direction, such that the first seal is in sealing contact with an exterior surface of the connector and where an air-flow path is established from an air-filled portion of the container through the venting hole and through the interface between the connector and coupling and past the second seal to a position distally of the connector inner lumen, thus providing a second air-vented position in which a pressure is equalized between the air-filled portion of the container and the inner lumen of the connector.

In an anal irrigation system as described above, the container will be pressurised due to air being pumped into the container, which is used to displace the liquid from the container. A tube connecting the container to an anal probe is attached to the container through a connector. The connector is provided with means for relieving the pressure, which is built up in the container by pumping of air into the container, these means typically being in the form of establishing an air-flow path, which comprises a venting hole. The pressure relieving means has the effect that as soon as the connector is moved axially, the seal will be positioned such that air from an air-filled portion of the container is allowed to be vented through the venting hole, either to the exterior of the container, or to a position distally of the connector.

A connector as described is able to relief the pressure built up in the container during use. This pressure relief happens by itself during the course of removing or detaching the connector from the container. Therefore, the user need not take any particular action in relation to pressure relief. Furthermore, the relief of the pressure allows for the connector to be removed without liquid spraying out of the container as the connector is removed. If the connector is attached to the container under the pressure, there is a high risk that when this connector is removed, then liquid will spray out from the connector opening in the container. This drawback is alleviated by the anal irrigation system as described herein.

In the following, whenever referring to a proximal end of end element described herein, the referral is to the end closest to the user of the system. Whenever referring to the distal end of an element, the referral is to the end opposite of that. In other words, the proximal end is the end upwards of the container and the distal end is the end downwards in the container.

The longitudinal direction is the direction from the distal to the proximal end. This may also be called the axial direction for the connector and the dip-tube. The axial direction corresponds to the direction of flow through the connector from the container and upwards through the inner lumen of the connector. A direction transversely to the axial direction is defined as being a radial direction.

An irrigation system typically comprises a reservoir or container for irrigation liquid, an anal probe and tubing connecting those two. The system will also include a pump for pumping the irrigation liquid into the intestines. In the anal irrigation system described herein, the pump is an air pump configured for pumping air into the container to pressurise it, and thus allow liquid for being displaced from the container. The pump may be a manual pump or an electric pump.

The container will have an air-filled portion at least during use. This portion will typically be the upper portion of the container, but other examples may be contemplated. This air-filled portion of the container will be in communication with an inlet from the pump, such that the pressure in this portion can increase and displace liquid from another portion of the container.

The anal probe typically comprises a stem in the form of a tubular part extending from the distal end towards the proximal end. The tip portion is positioned in the proximal end of the stem. The tip portion may be closed and provided with eyelets or alternatively, the tip portion may be open. The probe may comprise a connector in the distal end of the stem.

The connector has two portions, a distal portion, which is configured to be inserted into and removed from the container and a proximal portion configured for providing an interface for connecting a tube to the connector. The tube may be connected by a variety of ways, such as by welding or by detachably attaching a tube to the connector. The two portions may be separated by a flange providing an interface between the connector and an upper surface portion of the container (for example, the lid or the top of the connector inlet). The proximal portion may comprise a separate top part injection moulded on or snapped on to the proximal portion of the connector. The connector has an axially extending inner lumen configured for providing a liquid channel for liquid flow through the connector. The inner lumen and thus the liquid channel may extend form a liquid inlet in the distal portion of the connector to a liquid outlet in the proximal portion of the connector.

The connector is connected to the container via a coupling, and the connector is detachably attached to the container via the coupling.

The connector and coupling seals the connection between the two parts by a cooperation between these two elements, which jointly provides a sealing interface.

The connector is described as being configured to interact with the coupling to provide the connector with a first connected position in which the container can be pressurized by air being pumped into an air-filled portion of the container, and wherein the connector provides a liquid flow path from the container, through the inner lumen of the connector and to the exterior of the container. Thus, in this first connected position of the connector and coupling, the interface between the connector and coupling is sealed and air in an air-filled portion of the container is prevented from leaving the container allowing the container to be pressurized. This means that in this first connected position of the connector, the only passage out of the container is the liquid flow path through the inner lumen of the connector.

The connector is further described as being configured to interact with the coupling to provide the connector with a second air vented position, wherein an air-flow path is established from the air-filled portion of the container and through the venting hole in the coupling to either distally of the connector, or the exterior of the container, or both distally of the connector and the exterior of the container. In this second air vented position, the air-flow path allows the container to be de-pressurized, if the air-flow path goes to the exterior of the container. If the air-flow path goes to distally of the connector, then, in a first step, the pressure inside the container is still higher than outside, but the liquid remains inside the container, because the pressure is equalized between the air-filled portion of the container and the position distally of the connector. Therefore, the liquid will be prevented from flowing proximally through the inner lumen of the connector. The air vented position of the connector corresponds to either air venting to the exterior of the container or to internal air venting in the container between an air-filled portion of the container and a position inside the flow path, which is distally beyond the inner lumen of the connector.

The container may comprise a dip-tube providing the possibility of liquid flow from the bottom of the container to the connector. The dip-tube may extend from its proximal end, where it is attached to the coupling and towards its distal end close to the bottom of the container. The distal end should be below water level when the container is used for irrigation. The distal end may extend to the bottom of the container. The dip-tube may be integral with the coupling, such that the coupling and dip-tube are one element, with the coupling forming a proximal portion of this element and the dip-tube forming a distal portion of this element. Alternatively, the dip-tube may be connected to or connectable to the coupling.

An example of a sealing interface between the connector and the coupling is that the coupling comprises an upper protrusion (a proximal protrusion) and a lower protrusion (a distal protrusion) positioned on each side of a venting hole; i.e. positioned proximally of the venting hole and distally of the venting hole. The protrusions on the coupling extends radially inwards and may be in the form of a rounded ridge made of a soft, sealing material such as thermoplastic polyurethane, TPE, rubber.

The connector may comprise a bulge extending radially outwards. However, the connector may also have a smooth exterior surface, leaving the sealing to be on the coupling side of the interface between the coupling and connector.

As described above, the connector comprises at least two configurations, a first connected position and a second air-vented position. In the first connected position, the connector and coupling joint provide a sealing interface, such that the container can be pressurized meaning that air is prevented from leaving the container, and liquid can only leave the container through the liquid flow path established through the inner lumen of the connector. In the second air-vented position, air is allowed to be vented through the venting hole in the coupling to a position either distally of the connector, or the exterior of the container, but liquid is prevented from bypassing the connector.

As an example, the connector may also be in a third disconnected configuration, in which the connector is un-sealed to the container and can be freely removed from the container.

In the first connected position of the connector, a bulge on the connector may cooperate with an upper protrusion on the coupling to provide a seal preventing air as well as liquid from exiting the container, except for axially through the inner lumen of the connector. Alternatively, a proximal seal on the coupling is in interface with an exterior surface of the connector, such that a seal preventing air as well as liquid is prevented from exiting the container, except for axially through the inner lumen of the connector.

In the second position of the connector, the connector is axially displaced with respect to the coupling either upwards or downwards (proximally or distally). If the connector is provided with a bulge, then this may be positioned either below the upper protrusion or above the upper protrusion of the coupling. When the bulge is positioned axially displaced with respect to the upper protrusion, the sealing interface between these two elements is broken, and air is able to leave the air-filled portion of the container through the venting hole into a cavity between the connector and the coupling and leave the container. Alternatively, air may leave an air-filled portion of the container through the venting hole to a position distally of the connector

The container lid may comprise a lock and release-mechanism between the connector and the container lid. This lock and release-mechanism may be configured for providing an interface between an upper portion (in the proximal direction) of the distal part of the connector and the lock and release mechanism, whereas the interface between the coupling and connector is provided between a lower portion (in the distal direction) of the distal part of the connector and the coupling.

The lock and release mechanism provides the function of locking of the connector to the lid of the container and of releasing of the connector from the lid of the container. Locking of the connector to the lid may be advantageous because the container is pressurized and thus locking of the connector prevents the connector from being accidentally dislodged from the lid due to the pressure from the container. The releasing is an easy way to release a locked connector from the lid. The releasing may be configured for being done by users with poor hand dexterity, i.e. a simple push or pressure at a trigger or ejector is an advantage.

The connector parts may be Polypropylene or Polyethylene. It may also be made of recycled plastics.

In one example, the transition between the first position of the connector and the second position of the connector is done by pushing the connector downwards in a distal direction. In other words, the transition is a result of the connector being displaced downwards in a distal direction.

In one example, the transition between the first position of the connector and the second position of the connector is done by lifting the connector upwards in a proximal direction. In other words, the transition is a result of the connector being displaced upwards in a proximal direction.

Examples relate to the system comprising an ejector configured for cooperating with the connector for releasing the connector from the container.

Examples relate to the ejector comprising a lever arm.

Examples relate to the lock and release mechanism comprising a lock element with two snap arms cooperating with a skirt on the ejector. Examples relate to the lock element comprising three or four snap arms cooperating with a skirt on the ejector.

Examples relate to the lock and release mechanism comprising a lock element with a top part with an interior facing surface comprising downwards facing v-shaped ridges positioned with gaps between them and a bottom part with an interior facing surface comprising serrations. The distal portion of the connector comprises a stud, which is configured for cooperating with the v-shaped ridges and the serrated surface to function as a push to lock and push to release mechanism.

The top part and the bottom part may both be cylindrical and may be positioned proximally above the coupling in the lid of the container.

Examples relate to the stud on the connector having a hexagonal shape in cross-section. Upwards and downwards inclined surfaces on the stud may assist a rotational movement of the top part and bottom part as described in the following.

Examples relate to the lock and release-mechanism comprising a rotational part and a stationary part, the rotational part being configured to rotate with respect to the stationary part, wherein the stationary part holds a push-element configured to push downwards on the coupling and wherein the rotational part comprises a notch with a first portion configured for leaving room for rotation and a second portion, configured for holding a locking stud on the push-element.

Connecting the connector to a locked state and disconnecting to a released state is described in the following. In examples, a downwards push motion may be an initiation of disconnecting the connector from the container. The downwards push is the first step. In this configuration of the connector, the connector is in the second position and air is able to be vented from the container.

When the connector is released from pushing, the connector moves upwards due to a spring being positioned surrounding the top part and the bottom part, which cooperates with a skirt at the top of the lock and release mechanism. The skirt is in interface with the flange on the connector. During downwards pushing motion, the spring is tensioned, and when the connector is released, the spring will push upwards at the skirt and thus at the flange of the connector and lift it upwards.

During the downwards motion, the stud on the connector will have passed through one of the gaps on the inner surface of the top part and have entered into one of the troughs on the serrated surface. The serrated surface may be slightly askew such that it has an inclined surface and a more vertical surface meeting in an apex towards the top. However, the serrated surface may also be even, so that the inclination of the surfaces is the same on both sides of the apex. The stud will pass down the inclined surface - and during this motion, the top part and the bottom part are both rotated slightly corresponding to the length of the inclined surface in the radial direction. This means that when the connector is released and moves upwards (due to the spring) then the upward movement is stopped by one of the v-shaped protrusions on the top part. Again, the inclined surface of the v-shaped protrusion will assist in rotating the top part and the bottom part slightly. In this position, the connector is thus locked to the container - and the connector is in the first position, where the container is able to be pressurized

The next downwards pushing of the connector, will lead to the stud again connecting with the inclined surface of one of the serrations and a slight rotational movement of the top part and the bottom part. When the connector is pushed down as far as it can be pushed, air is able to be vented out of the container and the connector is thus in its second position. When the spring moves the connector upwards again, the stud will, due to the slight rotational movement of the top part and the bottom part, be aligned with a gap in the top part and will be able to pass through the gap. The connector is thus released from the lock and release mechanism.

In examples where the lock and release mechanism comprise an ejector, the connector is brought to a locked state by pushing it downwards. The flange on the connector will cooperate with a skirt on the ejector to push the skirt downwards and past the snap arms positioned radially inwards from the skirt. The snap arms may be positioned on a cylindrical lock element, which is positioned proximally above the coupling and positioned inside the skirt such that the skirt surrounds the cylindrical lock element. However, the lock element may also take other shapes, such as square, or a non-closed shape. The lock element includes the at least two snap arms and provides an element that keeps the snap arms in position, such that the only movement possible for the snap arms is radially outwards and inwards.

The connector is in these examples provided with a bulge configured for keeping the connector in a locked state when the bulge has moved downwards past the snap arms. The bulge may be the same bulge used in the air-relief mechanism. The bulge may be one annular bulge extending circumferentially around the outer surface of the connector. The bulge may also be two or more protruding elements positioned in discrete positions around the circumference of the outer surface of the connector. The bulge must be axially aligned with the snap arms and therefore, an annular bulge may be an advantage.

When the connector has been pushed so far down that the bulge on the connector has moved past the snap arms, the skirt will move downwards and cover the snap arm from the outside and thus prevent the snap arms from moving radially outwards. Therefore, the connector is prevented from accidentally or by itself move upwards out of the lid. In this position, the connector is in the first position and the container is able to be pressurized.

When the connector is to be released, the user pushes the proximal end of the lever arm of the ejector downwards and thus the distal end of the lever arm of the ejector, which includes the skirt, will move upwards - due to the seesawing motion over the ridge as described above. When the skirt has been lifted slightly, the snap arms are free from the skirt and able to move radially outwards. This will allow the bulge on the connector to move upwards past the snap arms and thus, the connector will be released. At the same time, a slight movement upwards will break the sealing interface between the connector and the coupling - and thus allow the container to be de-pressurized.

In examples the anal probe is provided with a retention part.

In examples, the retention part is in the form of an inflatable balloon.

In examples, the retention part is in the form of a foam element, in which case, the retention part is attached, connected or moulded to the proximal portion of the stem. The retention part increases in circumference from its distal portion towards the proximal portion. The largest circumference of the retention part will be between approximately 15 mm and 220 mm corresponding to a radial extent across the retention part in the transverse direction between 5 mm and 70 mm. Larger retention parts having a radial extent up to 75 mm may be contemplated.

The tubular part of the stem may a diameter of 5-10 mm.

In examples, the probe comprises a cone element attached to a stem. The cone element may have a proximal portion configured for being attached to a stem and flaring outwards from the attachment.

### Detailed Description of the Drawing

Initially, it shall be noted that the figures are schematic illustrations intended only to address the principles and functions of the anal probe described herein and are not to be considered limiting to the scope of the attached claims. Furthermore, the figures and particularly the individually illustrated elements are not necessarily to scale, neither individually nor in relation to each other.

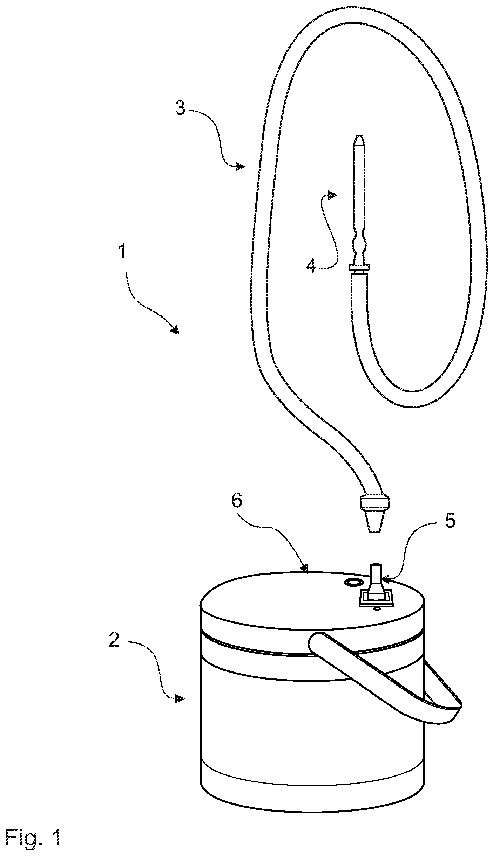

Figure 1 illustrates an anal irrigation system 1 as described herein. The system comprises a container for irrigation liquid 2, a tube 3 connected to an anal probe 4. A connector 5 connects the container 2 to the tube 3. In the example of the system illustrated in figure 1, the connector 5 is positioned in the lid 6 of the container. The container 2 may have other features, such as a handle, a scale, and so forth.

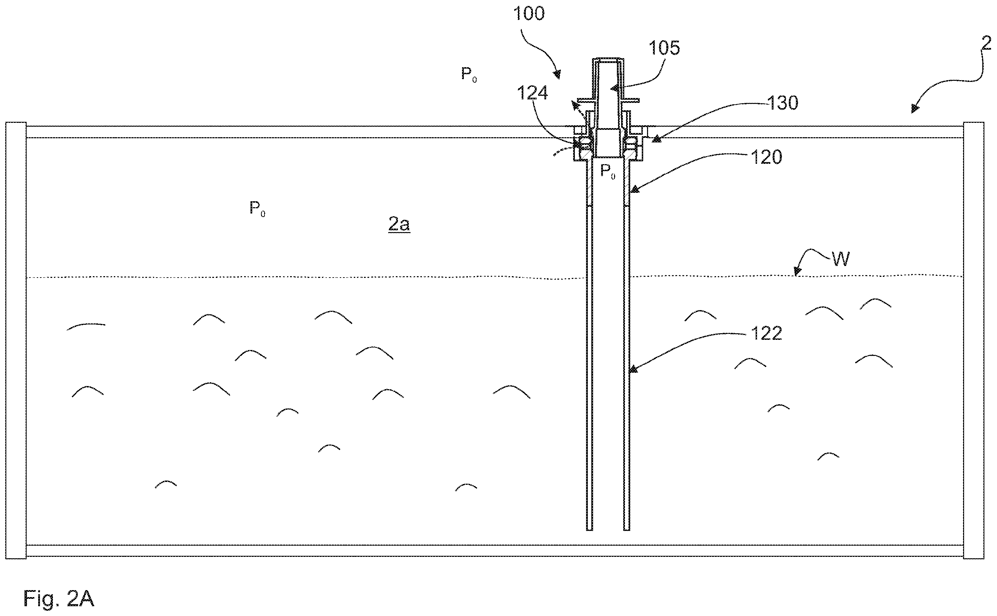

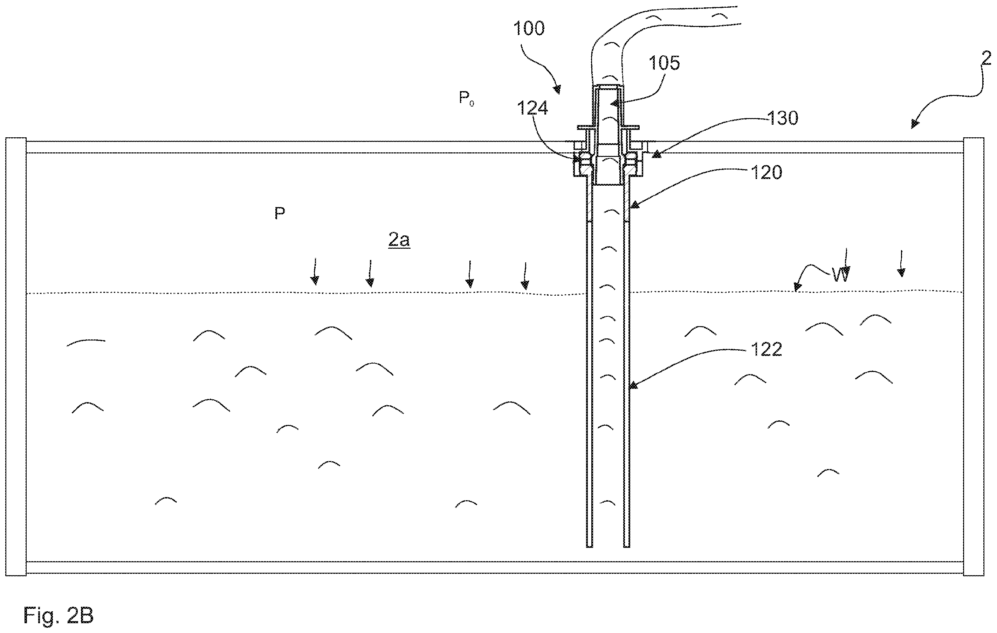

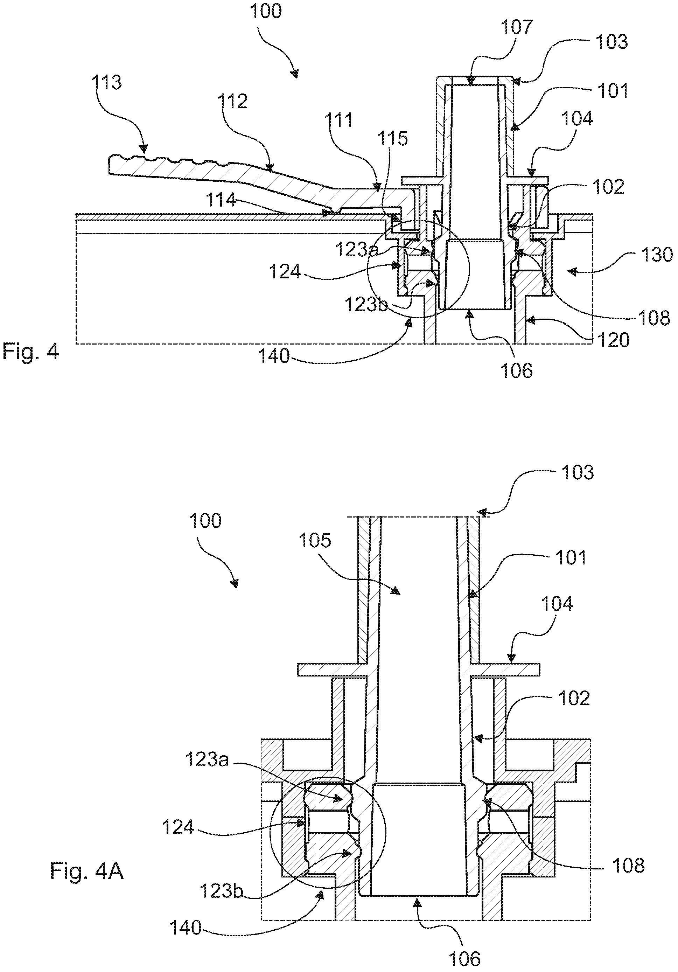

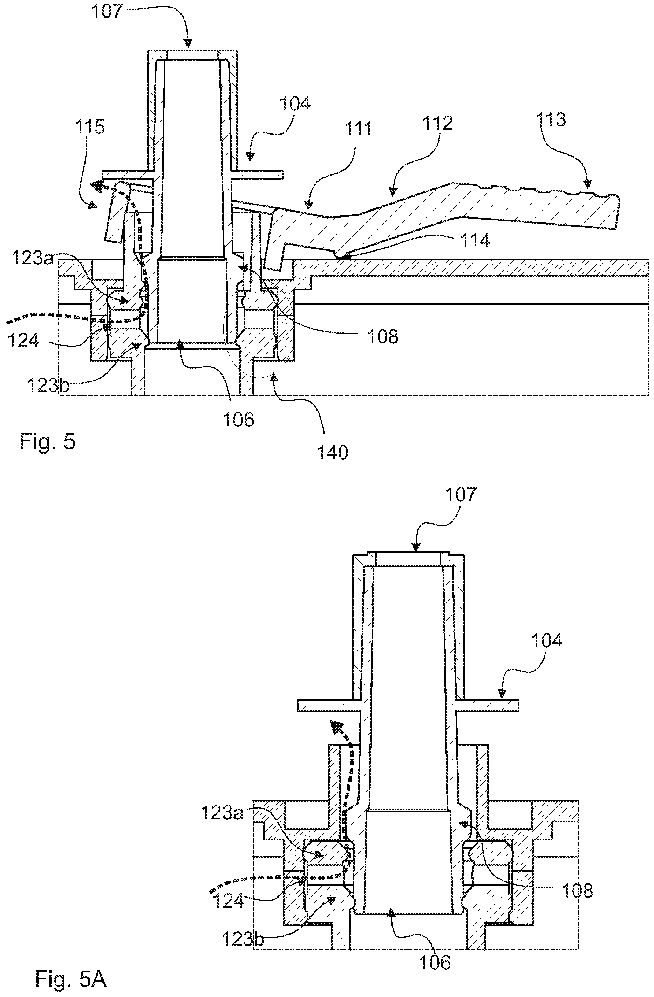

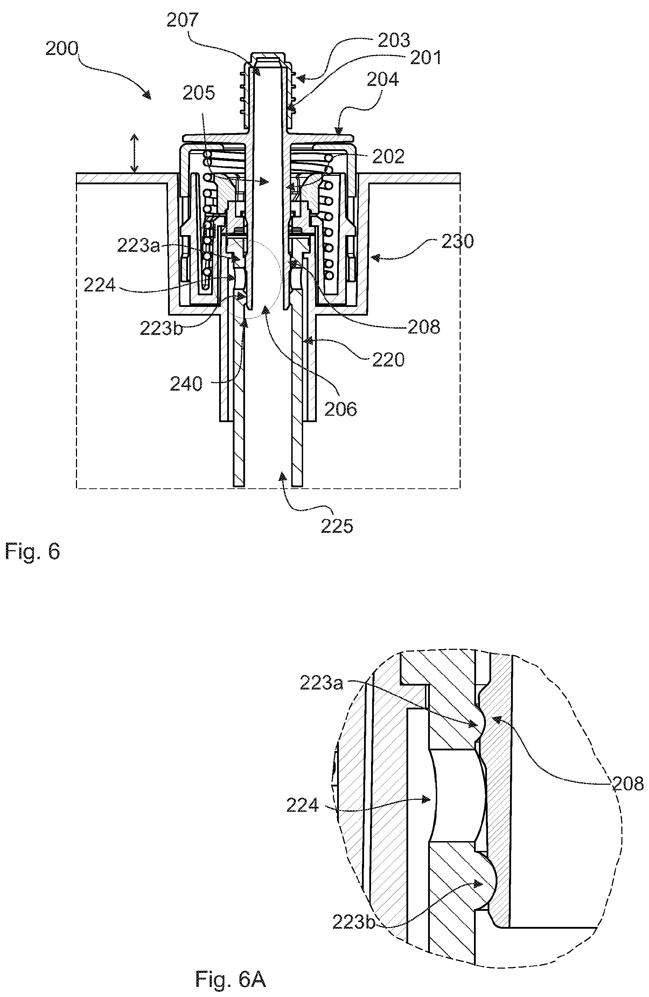

Figures 2A and 2B illustrate a container 2 for an irrigation system with a connector 100 as described herein. The connector 100 and the cooperation between the connector 100 and coupling 120 will be explained in more detail in figures 4 and 5. In figures 2A and 2B, the pressure relationship between the interior of the container and the exterior of the container (e.g. the ambience) is illustrated. In figure 2A, the connector 100 is in a second air-vented position, which is illustrated by the pressure everywhere being Po - both exterior of the container, corresponding to the ambience, interior of the container and inside the connector. Furthermore, it is illustrated by the dashed arrow that air can travel from the air-filled top portion of the container 2a to the exterior through the connector, because a seal 140 in an interface between the connector 100 and coupling 120 is not in sealed position. In figure 2B, the connector 100 is in a first connected position with respect to the coupling 120. In this position, the pressure interior of the container is P, due to air being pumped into the container by a pump (pump and inlet from the pump to the container is not shown). Thus, the air-filled portion of the container have an increased pressure P with respect to the exterior of the container. The seal 140 seals the interface between the connector 100 and the coupling 120 and thus prevents air from leaving a top portion 2a of the container. The pressure P will displace the liquid through a dip-tube 122 and outwards through the connector 100 and further through tubing 3 connected to the connector to a probe (which can be seen in figure 1). Thus, in this position, a liquid flow path through the inner lumen 105 of the connector is established. Following the irrigation procedure, the user may wish to disconnect the connector 100 prior to the container being emptied from liquid. In this situation, it is an advantage, if the container 2 can be air vented, e.g. by venting air from an air-filled top portion 2a of the container to the ambience as illustrated in figure 2A, such that the pressure inside a top portion of the container will be equal to the pressure Po exterior of the container. Thus, an air-flow path (indicated by the dashed arrow) between the air-filled portion 2a of the container, through the venting hole 124 in the coupling and out to the exterior of the container is established in the second air vented position of the connector. The connector illustrated in figures 4 and 5 and also the connector illustrated in figures 6, 7, and 8 will be able to establish a liquid flow path through the inner lumen (105, 205) of the connector and an air-flow path through the venting hole (124, 224) of the coupling as explained in relation to figures 2A and 2B.

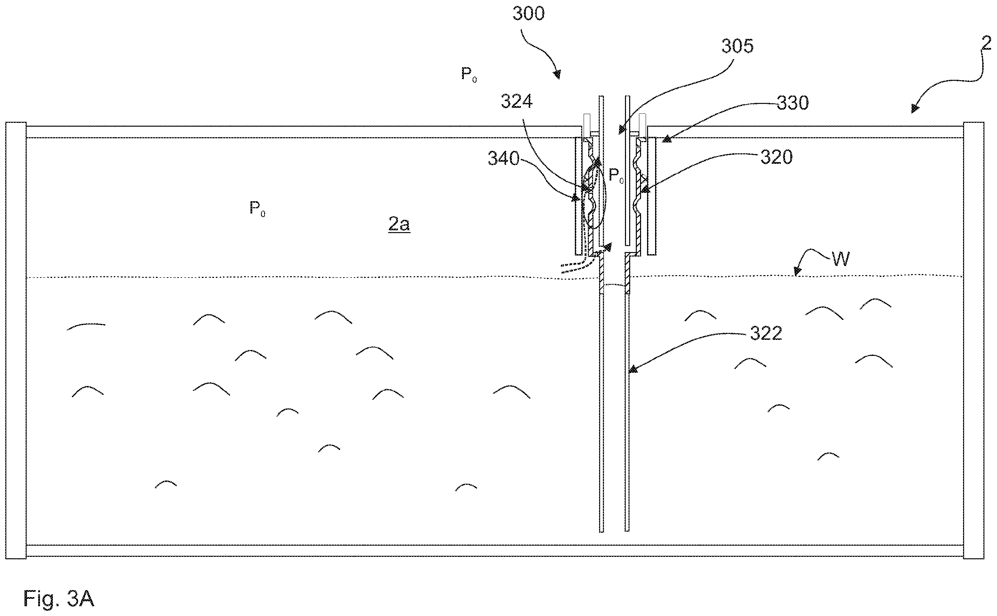

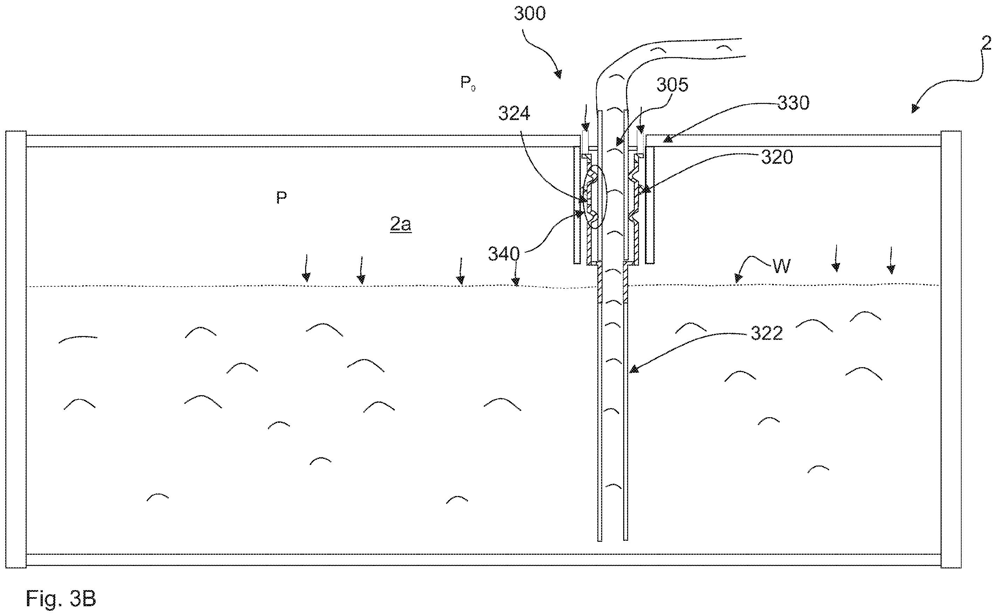

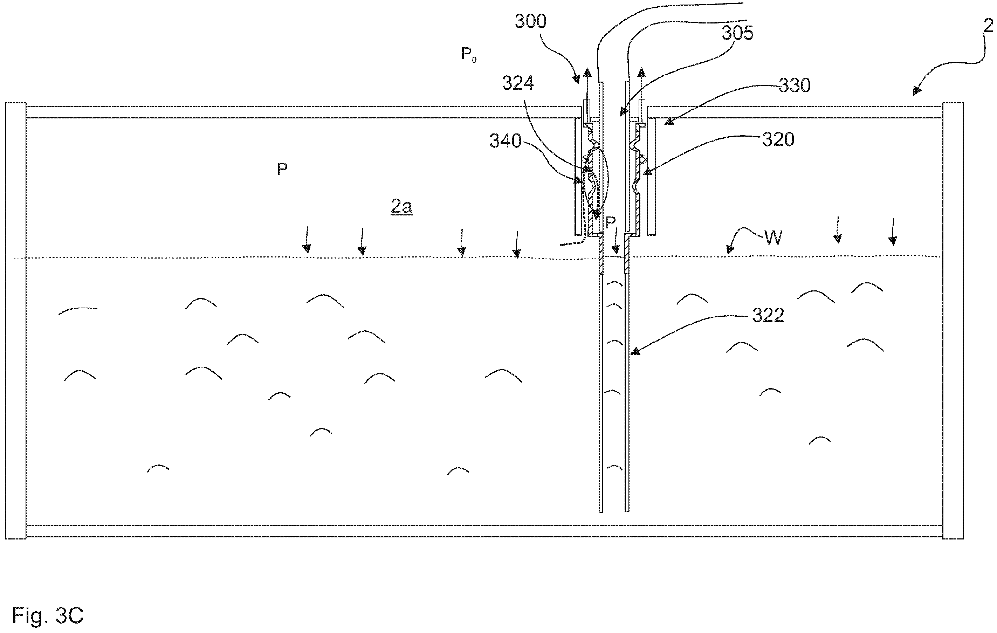

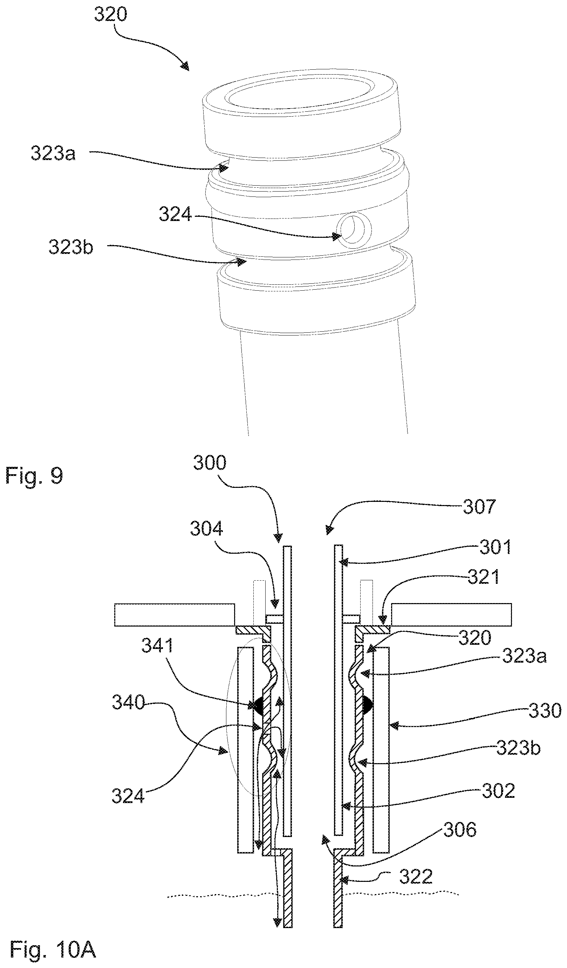

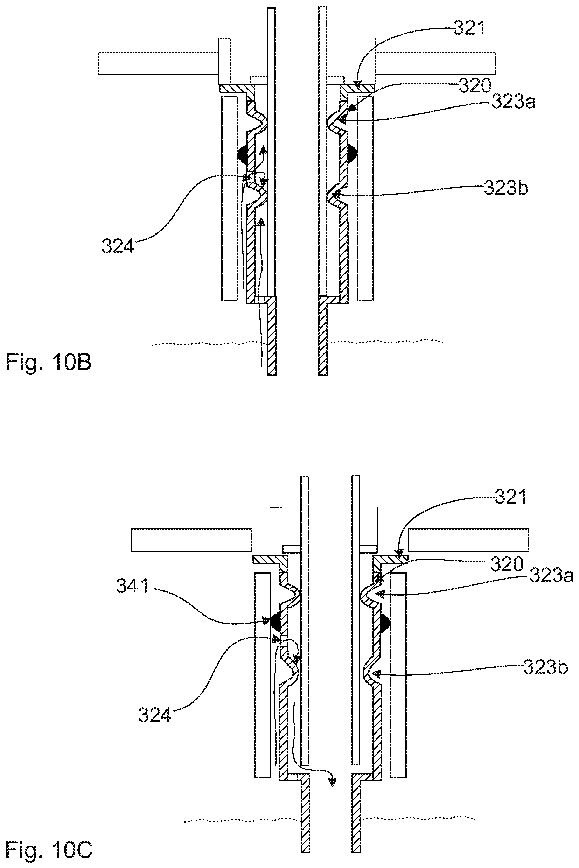

Figures 3A, 3B and 3C illustrate a container 2 for an irrigation system with a connector 300 as described herein. The connector 300 and the cooperation between the connector 300 and coupling 320 will be explained in more detail in figures 9 and 10A, 10B, 10C. In figures 3A, 3B and 3C, the pressure relationship between the interior of the container and the exterior of the container (e.g. the ambience) is illustrated. In Figure 3A, the connector 300 is in a "free position" just inserted loosely into the container. It is illustrated that the pressure everywhere is Po, interior of the container, exterior of the container and inside the connector. Furthermore, the arrows illustrate how the air inside the container can escape the container and also flow into the connector because a seal 340 in the interface between the coupling 320 and the connector 300 is not sealed. Figure 3B illustrate the connector 300 in a first connected position, in which the seal 340 between the connector 300 and the coupling 320 is sealed. In this position, the container can be pressurized by a pump (pump and inlet from pump to the container not shown) delivering air into the container. This is illustrated by the pressure P in an air-filled top portion 2a of the container being increased with respect to the pressure Po exterior of the container. The pressure P will lead the liquid inside the container to being displaced through a dip-tube 322, through the connector 300 and outwards of the container through a tubing 3 to a probe (see figure 1). In figure 3C, the connector is in a second air vented position; the seal 340 between the connector 300 and coupling 320 has been partly broken, such that an air-flow path is established between the air-filled portion of the container, through the venting hole 324 in the coupling and to a position distally of the connector. This is also indicated by the arrow. Therefore, liquid will no longer travel outwards through the connector but will stay inside the container. In this position of the connector 300, the user can safely disconnect the connector 300 from the container without risking liquid splashing out from the connector inlet 330 due to the overpressure inside the container. This step may be followed by a return of the connector to the free position illustrated in figure 3A, in which the air is vented from a top portion of the container. The connector illustrated in 9, 10A, 10B, and 10C will be able to establish an air-flow path in the container as explained in relation to figures 3A, 3B and 3C.

Turning now to a more detailed explanation of the establishing of a liquid flow path and an air-flow path, figures 4, 4A and 5, 5A illustrate an example of a connector 100 as described herein. Figure 4 illustrate the connector in a sealed position, figure 4A a close-up of the details and figure 5 illustrate the connector in a pressure equalizing position, figure 5A a close-up of the details. The connector 100 in this example comprises a proximal portion 101 and a distal portion 102. The container has a connector inlet portion 130 and a coupling 120 attached to the connector inlet portion. In this example, the coupling 120 is integral with a dip-tube 122 (see figure 2A, 2B) such that the coupling 120 is a top portion of an integral element having the dip-tube 122 extending into the container from the coupling. However, the coupling 120 may also be a separate element, which prior to use or during manufacturing is coupled to the dip-tube 122. The distal portion of the connector 102 is configured for being axially received in the coupling 120 and for being axially movable with respect to the coupling 120. The connector in this example has a top part 103 configured for creating an interface for attaching a tube connecting the connector to an anal probe, e.g. through a probe-connector. The connector top part 103 may in some examples be omitted in case the tube is directly connected to or connectable to the proximal portion 101 of the connector. The proximal portion 101 and the distal portion 102 of the connector is separated by a flange 104. An axially extending inner lumen 105 extends through the connector proximal and distal portion 101, 102 such that the axially extending inner lumen defines a liquid channel with a liquid inlet 106 and a liquid outlet 107.

The connector inlet 130 in container forms in the illustrated embodiment part of the lid 6 (only partly illustrated in figures 4 and 5). The connector inlet 130 extends downwards from the lid 6 and is configured for holding the coupling 120. The dip-tube is not illustrated in this embodiment, but it is configured for extending below the water level (also not illustrated) of the container as explained in relation to figure 2, 2A above.

The connector detachment 110 is in figure 2 a lever arm having three portions - a proximal horizontal portion 111, a middle-angled portion 112 and a distal horizontal portion 113. Between the proximal horizontal portion 111 and the middle-angled portion 112, a protrusion 114 extends towards the container lid 6. This protrusion is in the example illustrated as a ridge extending in a direction across the longitudinal direction of the lever arm, but other protrusions such as one or more bulges may be contemplated. The ridge 114 cooperates with the angled portion 112 to provide a seesaw mechanism allowing the lever to be tilted around the protrusion. An annular lift-element 115 is positioned so that it surrounds and encircles the distal portion 102 of the connector below the flange 104. Thereby the lift-element 115 will be able to lift the flange - and thereby the connector upwards from the container.

The coupling 120 comprises a seal, generally indicated as 140. In the illustrated example the seal 140 comprises a first proximal seal 123a towards the top, and a second distal seal 123b towards the container cavity. In the illustrated example, the first and second seal 123a, 123b are both in the form of sealing rings - however, other means may be contemplated, such as gaskets.

Between the two seals 123a, 123b the coupling 120 comprises a venting hole 124, which forms part of the air-flow path established in a second air vented position of the connector. The connector 100 comprises another part of the seal 140, also illustrated in the figures. This is a bulge 108 formed on a distal portion 102 of the connector. When the connector is in a connected and sealed position as shown in figure 4, the bulge 108 on the connector cooperates with the upper seal 123a on the coupling to prevent air and liquid from exiting the container - except from through the established liquid flow path, through the liquid inlet to the connector 106, the inner lumen 105 and out through the liquid outlet 107 of the connector.

In a second air vented position of the connector, air is able to be vented out from the container through the venting hole 124. This position is illustrated in figures 5 and 5A. From these figures, it can be seen that connector 100 is axially displaced upwards so that the bulge 108 is in a position above the upper seal 123a. However, the lower seal 123b is still in contact with the distal portion 102 of the connector and will keep the liquid from bypassing the connector. This means that the liquid can only leave the container through the liquid inlet 106 and the inner lumen 105 and exit through the liquid outlet 107. Only the pressurised air, which is in an air-filled portion 2a of the container, will be able to leave through the venting hole 124 and the air-flow pathway created. Once this air has been vented out, the connector can be lifted further upwards without any risk of liquid spraying up out of the coupling 120. Figure 5 illustrates the function of the lever arm 110, which seesaws over the ridge 114 so as to lift the flange 104 upwards by moving the ring-element 115 upwards. The user will push at the proximal portion 113 of the lever arm and thus move the connector upwards as illustrated.

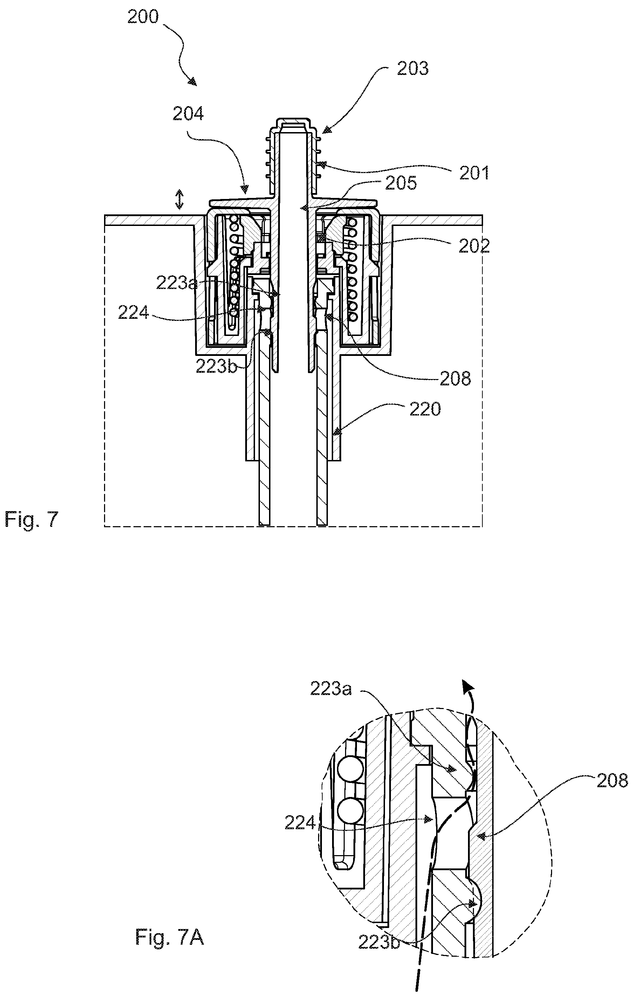

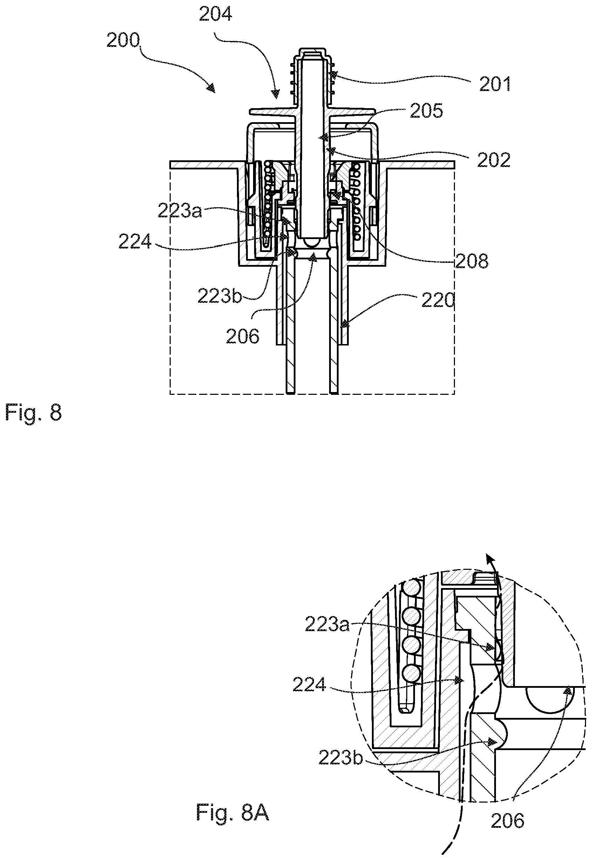

Figures 6, 6A, 7, 7A, 8, 8A illustrate another example of a connector 200 for a container as described herein. Figure 6 illustrate the connector in a first connected position, figure 6A a close-up of the details, figure 7 illustrate the connector in a second air vented position, figure 7A a close-up of the details and figure 8 illustrate the connector in a "free" position, figure 8A a close-up of the details.

The connector 200 is connectable to a coupling 220 attached to a connector inlet 230 of the container, as described in relation to figures 4 and 5. The connector has a proximal connector portion 201 and a distal connector portion 202. The interface between the coupling 220 and the connector 200 has a seal 240. This seal comprises a bulge 208 on the distal connector portion 202 configured for cooperating with proximal and distal seals 223a, 223b and a venting hole 224 to provide a first connected position and a second air-vented position of the connector. The connector is provided with a flange 204 providing an interface between the proximal portion 201 and the distal portion 202 of the connector. Furthermore, the connector is provided with a connector top part 203. The coupling 220 may be integral with or connectable to a dip-tube (not shown) as explained above.

In figure 6A, the close-up illustrates how the cooperation between the seals 223a, 223b and the bulge 208 ensures a first connected position of the connector, preventing air and liquid from bypassing the connector. Thus, in this first connected position, liquid flow is only possible through the liquid inlet 206, through the axially extending inner lumen 205 and out through the liquid outlet 207.

Figure 7 and 7A illustrate the connector of figure 6 in the second air vented position. The arrow at the top of the container illustrates by comparison to figure 6, how the connector is pressed downwards in a distal direction. In this configuration, the bulge 208 is positioned axially displaced with respect to the first proximal seal 223a. The close-up in figure 7A illustrates by the arrow how air can be vented through the venting hole 224 and the air-flow path established and bypass the connector in this position. Furthermore, it is illustrated how the distal seal 223b and the bulge 208 cooperates to prevent the liquid from bypassing the connector.

Figures 8 and 8A illustrate the connector of figures 6 and 7 when the connector is in a released or free position. In figure 8 it is illustrated how the liquid inlet 206 is above the venting hole 224 of the dip-tube. The bulge 208 is positioned above both the proximal 223a and the distal seal 223b. In the close-up in figure 8A it is illustrated how air is still able to bypass the connector and exit the container without exiting through the liquid inlet 206.

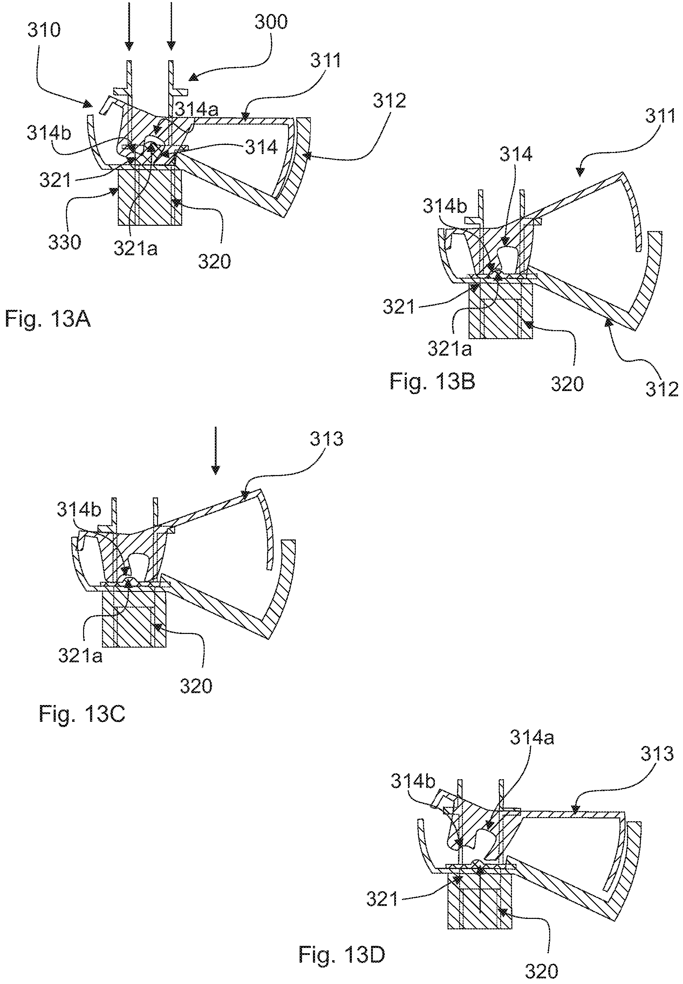

Figure 9 and 10A to 10C illustrate an example of a connector 300 in cooperation with a coupling 320, which in the illustrated example is integral with a dip-tube 322. Figure 9 illustrate a perspective view of a top portion of the coupling 320, whereas figures 10A to 10C illustrate cross-sectional views of the connector in three different positions. In figure 10A, the connector is about to be connected, meaning that the connector is still un-connected to the coupling 320. As explained with respect to the other examples, also this connector 300 has a proximal portion 301, a distal portion 302 and a flange 304 separating the two. The connector has an inner lumen 305 for liquid to pass through the connector from a connector inlet 306 to a connector outlet 307 at the top of the connector. The seal generally indicated as 340 in the interface between the connector 300 and the coupling 320 comprises a first proximal seal 323a and a second distal seal 323b. A venting hole 324 is positioned between the two seals 323a, 323b. The coupling 320 further comprises a push-element 321 configured for pushing downwards on the coupling so as to compress the coupling slightly into a sealed connection with the connector. The push-element 321 may be influenced by a trigger (see figure 13) - but it may also be pushed down through a cooperation with the connector, e.g. through a flange 304 on the connector. The push-element 321 is in the illustrated example illustrated as an element separate from the coupling 320, but it may also be provided integral with the coupling as a top portion thereof. In the illustrated example there is a further third seal 341 between the coupling and the connector inlet 330. This second seal will be explained in relation to figure 10B.

Figure 10B illustrate a first connected position of the connector 300 with respect to the coupling 320, i.e. in this position the opening into the container is sealed and the container can be pressurized. This corresponds to a first sealed configuration of the coupling. The coupling 320 is compressed slightly in the length (axial) direction, it can be seen that there is a close contact between the push-element 321 and the coupling 320. The first proximal seal 323a and the second distal seal 323b has moved radially inwards into connection with the connector 300 such that the interface between the connector and the coupling is sealed from air passing through this interface. The third seal 341 seals between the coupling 320 and the connector inlet 330 and thus prevents air from escaping this way. This is illustrated by the arrows always meeting obstacles from bypassing. In this sealed position of the connector, only liquid from the container can pass through the dip-tube 322 and from there through the connector inlet 306 into the inner lumen in the connector and finally leave the connector through the connector outlet 307 to a tube (not shown). Thus, in this position of the first and the second seal, the interface between coupling and connector is sealed and a liquid flow path is established through the inner lumen of the connector.

Figure 10C illustrate a second air vented position of the connector 300 with respect to the coupling 320. This corresponds to a second air vented configuration of the coupling. Comparing figure 10C with figure 10B illustrates that the coupling is slightly less compressed in figure 10C to such a degree that the distal seal 323b is no longer in engagement with the connector 320 - but rather air from an air-filled top portion of the container is able to pass through the venting hole 324 and past this distal seal to distally below connector inlet 306. It can be seen that the connector inlet 306 is lifted slightly with respect to the dip-tube 322 leaving room for air to move into the inner lumen 305 of the connector. Thus, an air-flow path is established between an air-filled portion of the container and distally of the connector. The proximal seal 323a as well as the second seal 341 are both still in sealing position with respect to the connector 320 and connector inlet 330, respectively. In this position the pressure is equalized such that the pressure inside the dip-tube 322, immediately below (or distally of) the connector 300 is the same as the pressure above the water level in the container. When the pressure is the same, the liquid in the container will no longer move upwards in the dip-tube but will stay where it is. This is explained in more detail in relation to figures 3A to 3C above.

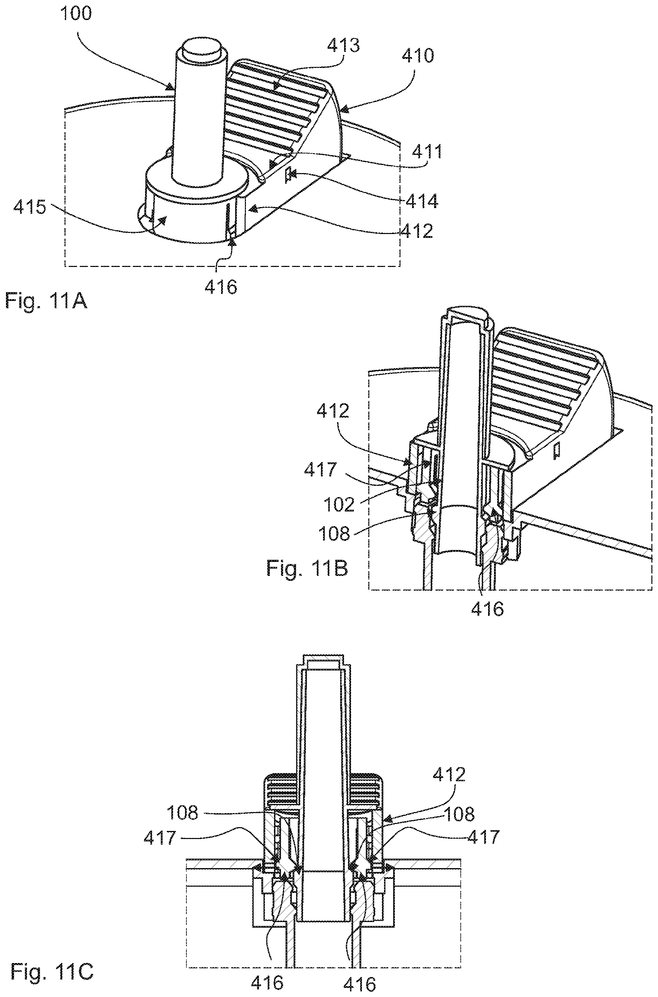

Figures 11A to 11C illustrate an example of a lock and release mechanism, which uses the principle of a lever arm as explained in relation to figures 4 and 5. In figures 11A to 11C, the connector 100 is similar to the one illustrated in figures 4 and 5, but the lever mechanism 410 is slightly different. The lever mechanism comprises a proximal portion 411 with a skirt 412 extending in the axial direction of the connector and at least partly surrounding a distal portion 102 of the connector. An upper surface of the skirt is configured to cooperate with the flange 105 of the connector so as to lift the connector in an upward direction from the container. The lever mechanism 410 seesaws over a ridge 414, so that when a user pushes the distal portion 413, the proximal portion 411 will be lifted upwards. In figure 11A, the proximal portion 411 is horizontal and the distal portion 413 is angled with respect to the proximal portion. This is the locked position of the lever arm mechanism 410. In this position, two snap arms 416 are prevented from moving radially outwards by the skirt 412 extending distally beyond the snap arms (figure 11B). The snap arms are each provided with a protuberance 417 toward the distal end. The protuberance 417 cooperates with the bulge 108 om the connector to keep the connector in a locked configuration. In figure 11C it is illustrated how the snap arms 316 are able to move radially outwards, when the skirt 312 is lifted upwards - due to the distal portion 313 of the lever being pushed downwards. When the snap arms are moved radially outwards, the bulge 108 of the connector is able to pass the snap arms and move upwards.

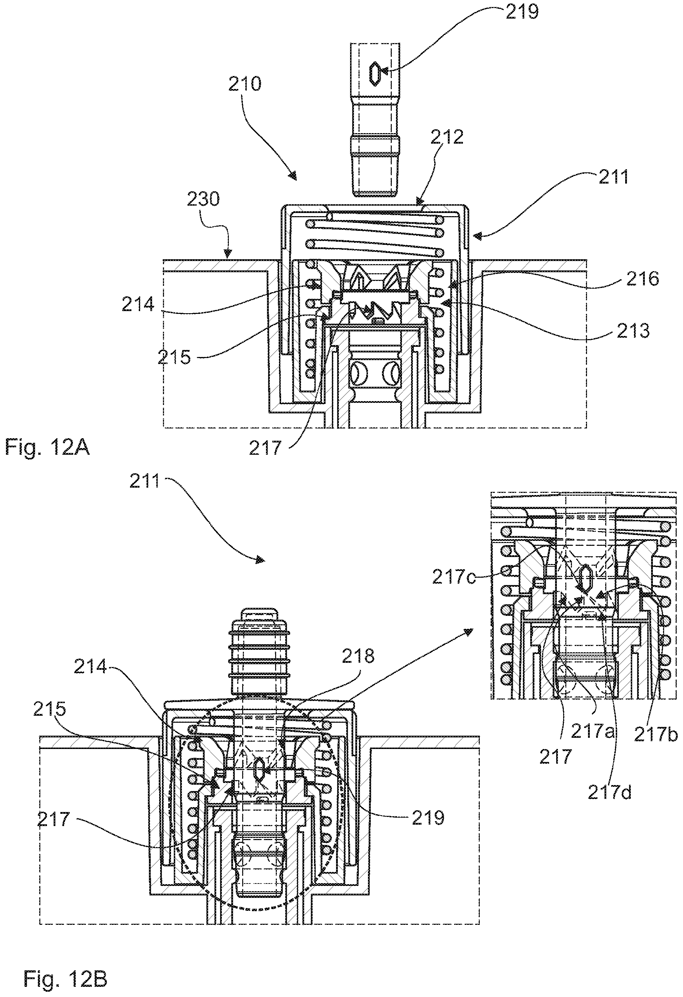

The lock and release mechanism (generally indicated as 210) of the connector in figures 6, 7 and 8 will now be explained in relation to figures 12A to 12H. As mentioned above, the lock and release-mechanism is positioned in the interface between the connector and the container at a top-surface 250 of the container. The lock and release mechanism is in this example similar to the function of a click ball-pen, i.e. push to lock and push again to release the connector 200 from the container.

The lock and release mechanism 210 comprises a skirt 211 with a central opening 212 through which, the distal portion 202 of the connector extends. This skirt is displaceable in an axial direction of the connector to an extent, which is controlled by a spring 213 surrounding the connector.

As the connector is pushed in an axial direction, the spring will be tensioned by the skirt. Furthermore, the underside of the skirt cooperates with a top part 214 of a lock element 216 to press it downwards into contact with a bottom part 215 of the lock element, which in turn causes the lock element 216 to rotate slightly due to a serrated surface 217 (see detail, figure 12B).

The top part 214 has a number of downwards facing, v-shaped ridges 218, which are used to catch a stud 219 on the distal part of the connector in the locked position.

The rotation of the top part 216 is ensured by the serrated surface 217, which is alternating between a vertical surface portion 217a and an inclined surface portion 217b connected at an apex 217c. This means, that when the protrusion is positioned at the apex 217c, it will, due to the contact with the inclined surface portion 217b, force the lock element 216 to rotate slightly (to the left in figure 12B) as the protrusion moves downwards. This movement is caused by a user pushing downwards on the connector.

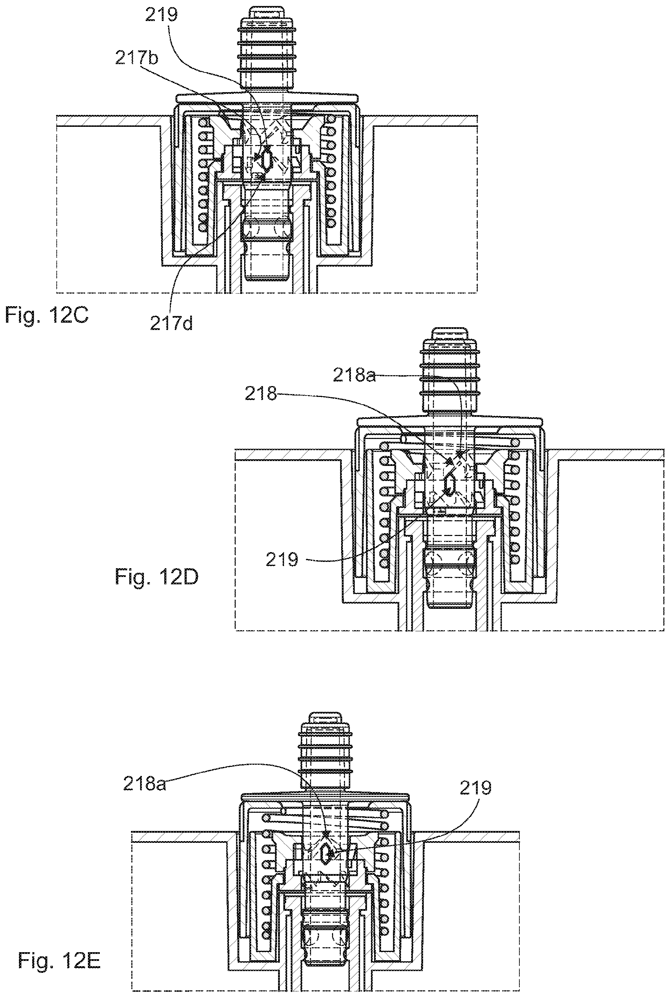

Figure 12C illustrates the connector system in a fully compressed position, where the connector is pushed as far down in the container, as is possible, and the spring is under maximum tension. The limit of the compression is due to the stud 219 reaching the trough 217d of the serrated surface following the stud 219 having been slid down the inclined surface 217b.

Figure 12D illustrates the connector in a position between the fully compressed and the locked position. The stud 219 is in contact with the v-shaped ridge 218, but has not yet reached the apex 218a of the v.

Figure 12E illustrates the connector in a locked position, where the stud 219 on the connector is in contact with the apex 218a of the v. The spring will urge the connector upwards, but the interaction between the stud 219 and the apex 218a of the v will prevent the connector from being released from the container.

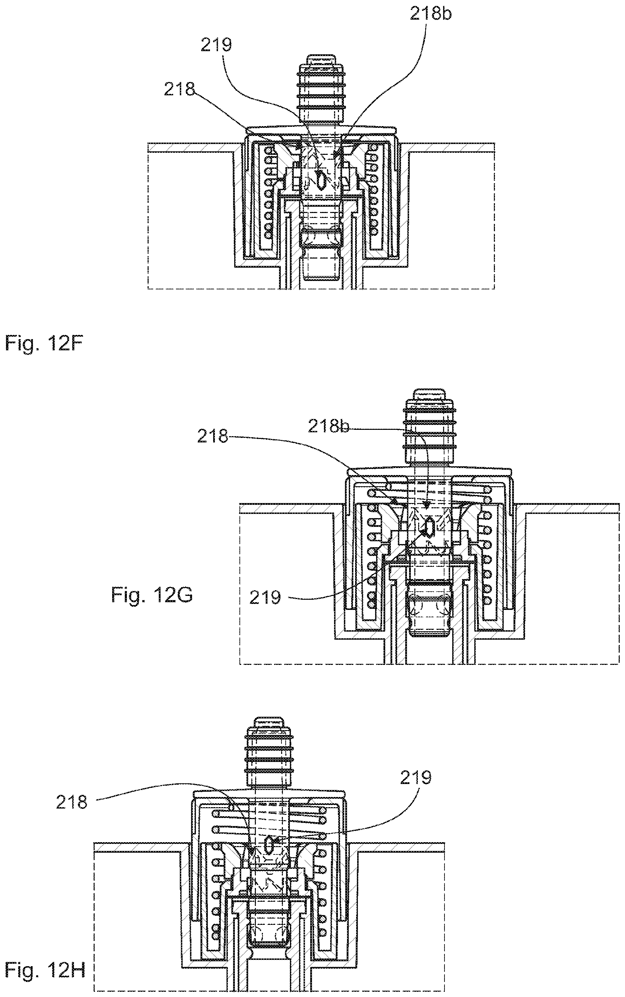

Figure 12F to 12H illustrates the connector in release. In figure 12F, the connector is pushed downwards and thus released from the v-shaped ridge 218. The push down is limited by the trough 217d of the serrated surface. The figures illustrate a gap 218b between the v-shaped ridges 218. In embodiments, there may be four v-shaped ridges with gaps between them. In embodiments there may be 6 v-shaped ridges with gaps between them. In figure 12G it is illustrated how the stud 219 can pass through the gap 218b between the v-shaped ridges 218, and in figure 12H the stud has passed through the gap and the connector can be released from the container.

Figures 13A to 13D illustrate a lock and release mechanism 310 in the form of a trigger for a connector 300 as illustrated in figures 9 and 10A, 10B and 10C. The lock and release mechanism functions through a cooperation between a rotatable part 311, which is rotatable with respect to a stationary part 312. The rotatable part 311 has a push-button 313 at a distal end and a notch 314 facing the push-element 321 in use. The notch has a first portion 314a configured for leaving room for rotation and a second portion 314b, configured for holding a locking stud 321a on the push-element.

Figures 13A illustrate a schematic drawing of the connector in an unlocked position. The connector 300 is lifted with respect to the coupling and the coupling 320 extends all the way to the top of the connector inlet 330 with the push-element 321 extending beyond the connector inlet. In this position, the notch 314 receives the locking stud 321a in the first portion 314a. Figure 13B illustrate a similar schematic drawing of the connector in a locked position. In the illustrated position, the flange 304 on the connector is in the locked position immediately above the rotatable part 311 of the lock and release mechanism. In other examples, the flange on the connector may be positioned lower and may be atop the push-element 321 as illustrated in figures 10A, 10B and 10C. The flange forms no part of the lock and release mechanism. In figure 13B, the push-element 321 is illustrated as being positioned in a lower position than in figure 13A. In this position this push-element has pushed the coupling 320 so that it is now slightly compressed as explained in relation to figure 10B. The rotatable part 311 has rotated with respect to the stationary part 312. The coupling 320 will, due to the resilience of the material, push the push-element upwards, but the locking stud 321a is caught under the second portion 314b of the notch as illustrated. This is due to the notch having rotated such that the second portion 314b of the notch is aligned with the locking stud 321a. Thus, the coupling and thereby the connector will be prevented from moving upwards. When the user wishes to release the connector 300, the user pushes the push-button 313 as indicated by the arrow and thus forces the rotatable part 311 to rotate. During this rotation, the push-element and the locking stud 321a will be released from the second portion 314b of the notch and will be able to move into the first portion 314a of the notch and move upwards. The resilience of the coupling material will function as a spring and force the push-element 321 upwards, as indicated by the arrow in figure 13D. Due to the friction between the connector 300 and the coupling 320 and push-element 321, the connector 300 will follow the coupling and push-element upwards. Thus the connector 320 will be released again.

Embodiments, and features of the various exemplary embodiments described in this application, may be combined with each other ("mixed and matched"), unless specifically noted otherwise. The scope of the invention is defined by the appended claims.

## Claims

1. An anal or stomal irrigation system (1) comprising
- a container (2) configured for containing irrigation liquid and configured for being pressurized,
- a pump configured for pumping air into the container so as to displace the liquid from the container,
- a connector (100, 200, 300) configured for connecting a tube to the container, the connector (100, 200, 300) comprising an inner lumen (105, 205, 305) extending through the connector
- the container comprising a coupling (120, 220, 320) comprising at least one seal (140, 240, 340) and a venting hole (124, 224, 324)
**characterised in that** the connector (100, 200, 300) is configured to interact with the coupling to provide the connector with
- a first connected position in which the container (2) can be pressurized by air being pumped into an air-filled portion of the container, and wherein the connector (100, 200, 300) provides a liquid flow path from the container, through the inner lumen (105, 205, 305) of the connector and to the exterior of the container, and
- a second air vented position, wherein an air-flow path is established from the air-filled portion of the container and through the venting hole (124, 224, 324) in the coupling either
∘ distally of the connector, or
∘ to the exterior of the container, or
∘ both distally of the connector and to the exterior of the container.

2. The anal or stomal irrigation system (1) as claimed in claim 1, wherein the coupling (120, 220, 320) comprises a first sealing and a second sealing positioned distally of the first sealing and wherein the venting hole (124, 224, 324) is positioned axially between the first sealing and the second sealing.

3. The anal or stomal irrigation system (1) as claimed in claim 1 or 2, wherein the air-filled portion of the container is an upper portion of the container.

4. The anal or stomal irrigation system (1) as in any of the preceding claims, wherein the container (2) comprises a dip-tube (122, 322) configured for providing flow of liquid from the bottom of the container to the connector.

5. The anal or stomal irrigation system as claimed in claim 4, wherein the dip-tube (122, 322) is integral with the coupling.

6. The anal or stomal irrigation system (1) as claimed in claim 4, wherein the dip-tube (122, 322) is connected to the coupling.

7. The anal or stomal irrigation system (1) as claimed in claim 1, wherein the seal (140, 240, 340) is in the form of one or more sealing rings.

8. The anal or stomal irrigation system (1) as claimed in claim 2, wherein the upper seal (123a) is in the form of a sealing ring and the lower seal (123b) is in the form of a sealing ring.

9. The anal or stomal irrigation system (1) as claimed in claim 1, wherein the seal (140, 240, 340) is in the form of one or more gaskets.

10. The anal or stomal irrigation system (1) as claimed in claim 2, wherein the upper and lower seals (123a, 123b) are in the form of gaskets.

11. The anal or stomal irrigation system (1) as claimed in any of the preceding claims, wherein the connector (100, 200, 300) comprises a bulge (108, 208) configured for cooperating with the seal(s) on the dip-tube to provide the sealing configurations.

12. The anal or stomal irrigation system (1) as claimed in any of the preceding claims, wherein the transition between the first connected position of the connector and the second air-vented position of the connector is a result of the connector (100, 200, 300) being displaced downwards in a distal direction.

13. The anal or stomal irrigation system (1) as claimed in any of the preceding claims, wherein the transition between the first connected position of the connector and the second air-vented position of the connector is a result of the connector (100, 200, 300) being displaced upwards in a proximal direction.

14. The anal or stomal irrigation system (1) as claimed in any of the preceding claims, wherein the connector (100, 200, 300) comprises an ejector configured for cooperating with the connector for releasing the connector from the container.

15. The anal or stomal irrigation system (1) as claimed in claim 14, wherein the ejector comprises a lever arm (110, 410).

16. The anal or stomal irrigation system (1) as claimed in any of the preceding claims, wherein the system comprises a lock and release mechanism (210, 310) for the connector.

17. The anal or stomal irrigation system (1) as claimed in claim 16, wherein the lock and release mechanism (210, 310) comprises a lock element with two snap arms cooperating with a skirt on the ejector.

18. The anal or stomal irrigation system (1) as claimed in claim 16, wherein the lock element comprises three or four snap arms cooperating with a skirt on the ejector

19. The anal or stomal irrigation system (1) as claimed in claim 16, wherein the lock and release mechanism (210, 310) comprising a lock element with a top part with an inwards facing surface comprising downwards facing v-shaped ridges positioned with gaps between them and a bottom part with an inwards facing surface comprising serrations and wherein the distal portion of the connector comprises a stud which is configured for cooperating with the v-shaped ridges and the serrated surface to function as a push to lock and push to release the lock and release mechanism.

20. The anal or stomal irrigation system (1) as claimed in claim 19, wherein the stud on the connector has a hexagonal shape in cross-section.

21. The anal or stomal irrigation system (1) as claimed in claim 16, wherein the lock and release mechanism (210, 310) comprises a rotational part and a stationary part, the rotational part being configured to rotate with respect to the stationary part, wherein the stationary part holds a push-element configured to push downwards on the coupling and wherein the rotational part comprises a notch with a first portion configured for leaving room for rotation and a second portion, configured for holding a locking stud on the push-element

22. The anal or stomal irrigation system (1) as claimed in any of the preceding claims, wherein the container comprises a lid and the lid comprises a connector inlet configured for holding the coupling.

23. A method for de-pressurizing a pressurized container in an anal or stomal irrigation system as claimed in any of the preceding claims, the method comprising the connector (100, 200, 300) being transitioned from a first connected position to a second air-vented position, whereby an air-flow path is established from the air-filled portion of the container and through the venting hole in the coupling, thereby de-pressurizing the connector (100, 200, 300).

24. The method as claimed in claim 23, wherein the transition between the first connected position of the connector and the second air-vented position of the connector is a result of the connector (100, 200, 300) being displaced downwards in a distal direction.

25. The method as claimed in claim 23, wherein the transition between the first connected position of the connector and the second air-vented position of the connector is a result of the connector (100, 200, 300) being displaced upwards in a proximal direction.

## Patentansprüche

1. Anal- oder Stoma-Irrigationssystem (1), umfassend
- einen Behälter (2), der dazu ausgelegt ist, Irrigationsflüssigkeit zu enthalten und mit Druck beaufschlagt zu werden,
- eine Pumpe, die dazu ausgelegt ist, Luft in den Behälter zu pumpen, um die Flüssigkeit aus dem Behälter zu verdrängen,
- einen Konnektor (100, 200, 300), der zur Verbindung eines Schlauchs mit dem Behälter ausgelegt ist, wobei der Konnektor (100, 200, 300) ein inneres Lumen (105, 205, 305) umfasst, das sich durch den Konnektor erstreckt
- wobei der Behälter eine Kupplung (120, 220, 320) umfasst, die mindestens eine Abdichtung (140, 240, 340) und ein Entlüftungsloch (124, 224, 324) umfasst
**dadurch gekennzeichnet, dass** der Konnektor (100, 200, 300) zum Zusammenwirken mit der Kupplung ausgelegt ist, um für den Konnektor Folgendes bereitzustellen
- eine erste verbundene Position, in der der Behälter (2) durch Luft, die in einen luftgefüllten Teil des Behälters gepumpt wird, mit Druck beaufschlagt werden kann, und wobei der Konnektor (100, 200, 300) einen Flüssigkeitsströmungsweg vom Behälter durch das innere Lumen (105, 205, 305) des Konnektors und zur Außenseite des Behälters bereitstellt, und
- eine zweite entlüftete Position, in der ein Luftströmungsweg vom luftgefüllten Teil des Behälters und durch das Entlüftungsloch (124, 224, 324) in der Kupplung hergestellt wird, entweder
∘ distal vom Konnektor oder
∘ zur Außenseite des Behälters oder
∘ sowohl distal vom Konnektor als auch zur Außenseite des Behälters.

2. Anal- oder Stoma-Irrigationssystem (1) nach Anspruch 1, wobei die Kupplung (120, 220, 320) eine erste Abdichtung und eine zweite Abdichtung, die distal von der ersten Abdichtung positioniert ist, umfasst und wobei das Entlüftungsloch (124, 224, 324) axial zwischen der ersten Abdichtung und der zweiten Abdichtung positioniert ist.

3. Anal- oder Stoma-Irrigationssystem (1) nach Anspruch 1 oder 2, wobei der luftgefüllte Teil des Behälters ein oberer Teil des Behälters ist.

4. Anal- oder Stoma-Irrigationssystem (1) nach einem der vorhergehenden Ansprüche, wobei der Behälter (2) ein Tauchrohr (122, 322) umfasst, das zum Bereitstellen eines Flüssigkeitsflusses vom Boden des Behälters zum Konnektor ausgelegt ist.

5. Anal- oder Stoma-Irrigationssystem nach Anspruch 4, wobei das Tauchrohr (122, 322) mit der Kopplung einstückig ist.

6. Anal- oder Stoma-Irrigationssystem (1) nach Anspruch 4, wobei das Tauchrohr (122, 322) mit der Kopplung verbunden ist.

7. Anal- oder Stoma-Irrigationssystem (1) nach Anspruch 1, wobei die Abdichtung (140, 240, 340) in Form eines oder mehrerer Dichtungsringe vorliegt.

8. Anal- oder Stoma-Irrigationssystem (1) nach Anspruch 2, wobei die obere Abdichtung (123a) in Form eines Dichtungsrings vorliegt und die untere Abdichtung (123b) in Form eines Dichtungsrings vorliegt.

9. Anal- oder Stoma-Irrigationssystem (1) nach Anspruch 1, wobei die Abdichtung (140, 240, 340) in Form einer oder mehrerer Dichtungen vorliegt.

10. Anal- oder Stoma-Irrigationssystem (1) nach Anspruch 2, wobei die obere und die untere Abdichtung (123a, 123b) in Form von Dichtungen vorliegen.

11. Anal- oder Stoma-Irrigationssystem (1) nach einem der vorhergehenden Ansprüche, wobei der Konnektor (100, 200, 300) eine Wölbung (108, 208) umfasst, die zum Zusammenwirken mit der/den Abdichtung(en) am Tauchrohr ausgelegt ist, um die Abdichtungskonfigurationen bereitzustellen.

12. Anal- oder Stoma-Irrigationssystem (1) nach einem der vorhergehenden Ansprüche, wobei der Übergang zwischen der ersten verbundenen Position des Konnektors und der zweiten entlüfteten Position des Konnektors darauf zurückzuführen ist, dass der Konnektor (100, 200, 300) nach unten in eine distale Richtung verschoben wird.

13. Anal- oder Stoma-Irrigationssystem (1) nach einem der vorhergehenden Ansprüche, wobei der Übergang zwischen der ersten verbundenen Position des Konnektors und der zweiten entlüfteten Position des Konnektors darauf zurückzuführen ist, dass der Konnektor (100, 200, 300) nach oben in eine proximale Richtung verschoben wird.

14. Anal- oder Stoma-Irrigationssystem (1) nach einem der vorhergehenden Ansprüche, wobei der Konnektor (100, 200, 300) eine Ausstoßvorrichtung umfasst, die zum Zusammenwirken mit dem Konnektor ausgelegt ist, um den Konnektor vom Behälter zu lösen.

15. Anal- oder Stoma-Irrigationssystem (1) nach Anspruch 14, wobei die Ausstoßvorrichtung einen Hebelarm (110, 410) umfasst.

16. Anal- oder Stoma-Irrigationssystem (1) nach einem der vorhergehenden Ansprüche, wobei das System einen Verriegelungs- und Freigabemechanismus (210, 310) für den Konnektor umfasst.

17. Anal- oder Stoma-Irrigationssystem (1) nach Anspruch 16, wobei der Verriegelungs- und Freigabemechanismus (210, 310) ein Verriegelungselement mit zwei Schnapparmen umfasst, die mit einer Schürze an der Ausstoßvorrichtung zusammenarbeiten.

18. Anal- oder Stoma-Irrigationssystem (1) nach Anspruch 16, wobei das Verriegelungselement drei oder vier Schnapparme umfasst, die mit einer Schürze an der Ausstoßvorrichtung zusammenarbeiten.

19. Anal- oder Stoma-Irrigationssystem (1) nach Anspruch 16, wobei der Verriegelungs- und Freigabemechanismus (210, 310) ein Verriegelungselement mit einem oberen Teil mit einer nach innen gerichteten Oberfläche umfasst, die nach unten gerichtete V-förmige Rillen mit Lücken dazwischen umfasst, und mit einem unteren Teil mit einer nach innen gerichteten Oberfläche, die Zacken umfasst, und wobei der distale Teil des Konnektors einen Bolzen umfasst, der dazu ausgelegt ist, mit den V-förmigen Rillen und der gezackten Oberfläche zusammenzuwirken, um als Schieber zum Verriegeln und Schieben zur Freigabe des Verriegelungs- und Freigabemechanismus zu fungieren.

20. Anal- oder Stoma-Irrigationssystem (1) nach Anspruch 19, wobei der Bolzen am Konnektor im Querschnitt eine sechseckige Form aufweist.

21. Anal- oder Stoma-Irrigationssystem (1) nach Anspruch 16, wobei der Verriegelungs- und Freigabemechanismus (210, 310) einen rotierenden Abschnitt und einen stationären Abschnitt umfasst, wobei der rotierende Abschnitt dazu ausgelegt ist, sich bezüglich des stationären Abschnitts zu drehen, wobei der stationäre Abschnitt ein Schubelement hält, das dazu ausgelegt ist, nach unten auf die Kupplung zu drücken, und wobei der rotierende Abschnitt eine Kerbe mit einem ersten Teil, der Platz für Rotationen lässt, und mit einem zweiten Teil umfasst, der dazu ausgelegt ist, einen Verriegelungsbolzen am Schubelement zu halten.

22. Anal- oder Stoma-Irrigationssystem (1) nach einem der vorhergehenden Ansprüche, wobei der Behälter einen Deckel umfasst und der Deckel einen Konnektoreinlass umfasst, der zum Halten der Kupplung ausgelegt ist.

23. Verfahren zur Druckentlastung eines druckbeaufschlagten Behälters in einem Anal- oder Stoma-Irrigationssystem nach einem der vorhergehenden Ansprüche, wobei das Verfahren umfasst, dass der Konnektor (100, 200, 300) von einer ersten verbundenen Position in eine zweite entlüftete Position überführt wird, wobei ein Luftströmungsweg vom luftgefüllten Teil des Behälters und durch das Entlüftungsloch in der Kupplung hergestellt wird, wodurch der Konnektor (100, 200, 300) druckentlastet wird.

24. Verfahren nach Anspruch 23, wobei der Übergang zwischen der ersten verbundenen Position des Konnektors und der zweiten entlüfteten Position des Konnektors darauf zurückzuführen ist, dass der Konnektor (100, 200, 300) nach unten in eine distale Richtung verschoben wird.

25. Verfahren nach Anspruch 23, wobei der Übergang zwischen der ersten verbundenen Position des Konnektors und der zweiten entlüfteten Position des Konnektors darauf zurückzuführen ist, dass der Konnektor (100, 200, 300) nach oben in eine proximale Richtung verschoben wird.

## Revendications

1. Système d'irrigation anale ou stomiale (1) comprenant
- un récipient (2) conçu pour contenir un liquide d'irrigation et conçu pour être mis sous pression,
- une pompe conçue pour pomper de l'air dans le récipient de manière à déplacer le liquide depuis le récipient,
- un raccord (100, 200, 300) conçu pour raccorder un tube au récipient, le raccord (100, 200, 300) comprenant une lumière interne (105, 205, 305) s'étendant à travers le raccord
- le récipient comprenant un accouplement (120, 220, 320) comprenant au moins un joint (140, 240, 340) et un trou de mise à l'air libre (124, 224, 324)
**caractérisé en ce que** le raccord (100, 200, 300) est conçu pour interagir avec l'accouplement pour fournir au raccord
- une première position raccordée dans laquelle le récipient (2) peut être mis sous pression par de l'air pompé dans une partie remplie d'air du récipient, et dans laquelle le raccord (100, 200, 300) fournit un trajet d'écoulement de liquide depuis le récipient, à travers la lumière interne (105, 205, 305) du raccord et vers l'extérieur du récipient, et
- une seconde position de mise à l'air libre, dans laquelle un trajet d'écoulement d'air est établi à partir de la partie remplie d'air du récipient et à travers le trou de mise à l'air libre (124, 224, 324) dans l'accouplement soit
∘ distalement par rapport au raccord, soit
∘ vers l'extérieur du récipient, soit
∘ à la fois distalement par rapport au raccord et vers l'extérieur du récipient.

2. Système d'irrigation anale ou stomiale (1) selon la revendication 1, dans lequel l'accouplement (120, 220, 320) comprend un premier joint et un second joint positionné distalement par rapport au premier joint et dans lequel le trou de mise à l'air libre (124, 224, 324) est positionné axialement entre le premier joint et le second joint.

3. Système d'irrigation anale ou stomiale (1) selon la revendication 1 ou 2, dans lequel la partie remplie d'air du récipient est une partie supérieure du récipient.

4. Système d'irrigation anale ou stomiale (1) selon l'une quelconque des revendications précédentes, dans lequel le récipient (2) comprend un tube plongeur (122, 322) conçu pour fournir un écoulement de liquide depuis le fond du récipient jusqu'au raccord.

5. Système d'irrigation anale ou stomiale selon la revendication 4, dans lequel le tube plongeur (122, 322) est solidaire de l'accouplement.

6. Système d'irrigation anale ou stomiale (1) selon la revendication 4, dans lequel le tube plongeur (122, 322) est raccordé à l'accouplement.

7. Système d'irrigation anale ou stomiale (1) selon la revendication 1, dans lequel le joint (140, 240, 340) se présente sous la forme d'une ou de plusieurs bagues d'étanchéité.

8. Système d'irrigation anale ou stomiale (1) selon la revendication 2, dans lequel le joint supérieur (123a) se présente sous la forme d'une bague d'étanchéité et le joint inférieur (123b) se présente sous la forme d'une bague d'étanchéité.

9. Système d'irrigation anale ou stomiale (1) selon la revendication 1, dans lequel le joint (140, 240, 340) se présente sous la forme d'un ou de plusieurs joints plats.

10. Système d'irrigation anale ou stomiale (1) selon la revendication 2, dans lequel les joints supérieur et inférieur (123a, 123b) se présentent sous la forme de joints plats.

11. Système d'irrigation anale ou stomiale (1) selon l'une quelconque des revendications précédentes, dans lequel le raccord (100, 200, 300) comprend un renflement (108, 208) conçu pour coopérer avec le ou les joints sur le tube plongeur pour fournir les configurations d'étanchéité.

12. Système d'irrigation anale ou stomiale (1) selon l'une quelconque des revendications précédentes, dans lequel le passage entre la première position raccordée du raccord et la seconde position mise à l'air libre du raccord est le résultat du déplacement du raccord (100, 200, 300) vers le bas dans une direction distale.

13. Système d'irrigation anale ou stomiale (1) selon l'une quelconque des revendications précédentes, dans lequel le passage entre la première position raccordée du raccord et la seconde position mise à l'air libre du raccord est le résultat du déplacement du raccord (100, 200, 300) vers le haut dans une direction proximale.

14. Système d'irrigation anale ou stomiale (1) selon l'une quelconque des revendications précédentes, dans lequel le raccord (100, 200, 300) comprend un éjecteur conçu pour coopérer avec le raccord pour libérer le raccord du récipient.

15. Système d'irrigation anale ou stomiale (1) selon la revendication 14, dans lequel l'éjecteur comprend un bras de levier (110, 410).

16. Système d'irrigation anale ou stomiale (1) selon l'une quelconque des revendications précédentes, dans lequel le système comprend un mécanisme de verrouillage et de libération (210, 310) pour le raccord.

17. Système d'irrigation anale ou stomiale (1) selon la revendication 16, dans lequel le mécanisme de verrouillage et de libération (210, 310) comprend un élément de verrouillage doté de deux bras d'encliquetage coopérant avec une jupe sur l'éjecteur.

18. Système d'irrigation anale ou stomiale (1) selon la revendication 16, dans lequel l'élément de verrouillage comprend trois ou quatre bras d'encliquetage coopérant avec une jupe sur l'éjecteur.

19. Système d'irrigation anale ou stomiale (1) selon la revendication 16, dans lequel le mécanisme de verrouillage et de libération (210, 310) comprend un élément de verrouillage doté d'une partie supérieure dotée d'une surface tournée vers l'intérieur comprenant des nervures en forme de V tournées vers le bas positionnées avec des espaces entre elles et d'une partie inférieure dotée d'une surface tournée vers l'intérieur comprenant des dentelures et dans lequel la partie distale du raccord comprend un goujon qui est conçu pour coopérer avec les nervures en forme de V et la surface dentelée pour fonctionner comme un mécanisme de verrouillage et de déverrouillage par pression.

20. Système d'irrigation anale ou stomiale (1) selon la revendication 19, dans lequel le goujon sur le raccord a une forme hexagonale en coupe transversale.

21. Système d'irrigation anale ou stomiale (1) selon la revendication 16, dans lequel le mécanisme de verrouillage et de libération (210, 310) comprend une partie rotative et une partie stationnaire, la partie rotative étant conçue pour tourner par rapport à la partie stationnaire, dans lequel la partie stationnaire maintient un élément de poussée conçu pour pousser vers le bas sur l'accouplement et dans lequel la partie rotative comprend une encoche dotée d'une première partie conçue pour laisser de la place pour la rotation et d'une seconde partie, conçue pour maintenir un goujon de verrouillage sur l'élément de poussée.

22. Système d'irrigation anale ou stomiale (1) selon l'une quelconque des revendications précédentes, dans lequel le récipient comprend un couvercle et le couvercle comprend une entrée de raccord conçue pour maintenir l'accouplement.

23. Procédé de mise hors pression d'un récipient mis sous pression dans un système d'irrigation anale ou stomiale selon l'une quelconque des revendications précédentes, le procédé comprenant le passage du raccord (100, 200, 300) d'une première position raccordée à une seconde position mise à l'air libre, moyennant quoi un trajet d'écoulement d'air est établi à partir de la partie remplie d'air du récipient et à travers le trou de mise à l'air libre dans l'accouplement, mettant de ce fait le raccord (100, 200, 300) hors pression.

24. Procédé selon la revendication 23, dans lequel le passage entre la première position raccordée du raccord et la seconde position mise à l'air libre du raccord est le résultat du déplacement du raccord (100, 200, 300) vers le bas dans une direction distale.

25. Procédé selon la revendication 23, dans lequel le passage entre la première position raccordée du raccord et la seconde position mise à l'air libre du raccord est le résultat du déplacement du raccord (100, 200, 300) vers le haut dans une direction proximale.
